# FASCICULE DE BREVET EUROPEEN

(11) **EP 2 456 743 B1**
(45) Date de publication et mention de la délivrance du brevet: **06.05.2015**
(21) Numéro de dépôt: 10745372.2
(22) Date de dépôt: 29.06.2010
(51) Int. Cl.: C07C 45/52, C07C 51/25, C07C 47/22, C07C 57/04, C07C 57/055, C07C 51/235, C07C 51/43, C07C 51/44, C07C 51/48, C08F 20/06, A61L 15/24

(54) **PROCEDE DE FABRICATION D'ACIDE ACRYLIQUE BIO-RESSOURCE A PARTIR DE GLYCEROL**
VERFAHREN ZUR HERSTELLUNG VON ACRYLSÄURE AUS GLYCERIN AUS BIORESSOURCEN
METHOD FOR PRODUCING BIORESOURCED ACRYLIC ACID FROM GLYCEROL

(30) Priorité: 22.07.2009 FR 0955111
(43) Date de publication de la demande: 30.05.2012
(73) Titulaire: Arkema France, 92700 Colombes (FR)
(72) Inventeur: FAUCONET, Michel, F-57730 Valmont (FR)
(74) Mandataire: Bonnel, Claudine
(86) Numéro de dépôt international: PCT/FR2010/051361
(87) Numéro de publication internationale: WO 2011/010035

(56) Documents cités:
- FR-A- 2 909 999
- US-A1- 2001 020 111
- US-A1- 2007 219 521

## Description

La présente invention vise un procédé de fabrication d'un acide acrylique bio-ressourcé à partir de glycérol comme matière première, le terme acide bio-ressourcé indiquant que l'acide acrylique est essentiellement fondé sur une source de carbone d'origine naturelle.

L'acide acrylique est une matière première très importante qui peut être utilisée directement pour obtenir un polymère d'acide acrylique ou, après estérification avec des alcools, pour produire un polymère de l'ester correspondant. Ces polymères sont utilisés tels quels ou en tant que copolymères dans des domaines aussi variés que l'hygiène (par exemple dans la production de superabsorbants), les détergents, les peintures, les vernis, les adhésifs, le papier, les textiles, le cuir, etc...

Les industriels ont développé depuis des décennies des procédés de synthèse de l'acide acrylique.

Une première génération utilisait comme matière première des composés à triple liaison, de type acétylénique, que l'on faisait réagir avec un mélange de monoxyde de carbone et d'eau en présence d'un catalyseur à base de nickel.

La deuxième génération de procédés, aujourd'hui principal procédé de production d'acide acrylique, est basée sur l'oxydation de propylène et/ou de propane. Ces matières premières sont issues du pétrole ou de gaz natuel, et par conséquent l'acide acrylique est constitué à partir d'une matière première carbonée fossile non renouvelable. En outre, les procédés d'extraction, purification et synthèse des matières premières ainsi que les processus de destruction en fin de cycle des produits finis fabriqués à base de ces matières premières fossiles génèrent du dioxyde de carbone, ce dernier étant un sous-produit direct des réactions d'oxydation du propylène en acroléine puis d'acroléine en acide acrylique. Tout ceci contribue à l'accroissement de la concentration des gaz à effets de serre dans l'atmosphère. Dans le cadre des engagements de la plupart des pays industrialisés à réduire les émissions de gaz à effet de serre, il apparaît particulièrement important de fabriquer de nouveaux produits à base de matière première renouvelable contribuant à réduire ces effets environnementaux.

Depuis quelques années, les industriels ont orienté leurs travaux de recherche et de développement sur des procédés de synthèse dits « bio-ressourcés » utilisant des matières premières naturelles renouvelables. En effet, pour limiter l'impact écologique des procédés de production classiques, des procédés alternatifs à partir de matières premières végétales non fossiles ont été développés récemment. Il s'agit par exemple de procédés utilisant comme matière première l'acide 2-hydroxypropionique (acide lactique), obtenu par fermentation de glucose ou de mélasse provenant de la biomasse. Il s'agit également de procédés à partir de glycérol (appelé aussi glycérine), issu de la méthanolyse des huiles végétales en même temps que les esters méthyliques qui sont, eux, employés notamment comme carburants ou combustibles dans le gazole et le fioul domestique. Ce glycérol est un produit naturel qui jouit d'une aura "verte", il est disponible en grande quantité et peut être stocké et transporté sans difficulté.

La méthanolyse des huiles végétales ou des graisses animales peut s'effectuer selon différents procédés bien connus , notamment en utilisant une catalyse homogène telle que la soude ou le méthylate de sodium en solution dans le méthanol, ou en utilisant une catalyse hétérogène. On pourra se reporter sur ce sujet à l'article de D. Ballerini et al. dans l'Actualité Chimique de nov-déc 2002.

Les procédés utilisant l'acide hydroxypropionique comme matière première ont un inconvénient majeur du point de vue économique. Ils mettent en jeu une réaction de fermentation qui est réalisée nécessairement en condition très diluée dans l'eau. Pour obtenir l'acide acrylique, une quantité très importante d'eau doit être éliminée par distillation, au prix d'un coût énergétique très important. Par ailleurs, l'énergie dépensée pour séparer l'eau, énergie produite à partir de matière fossile, vient dégrader fortement l'avantage initial de produire l'acide acrylique à partir de cette matière première bio-ressourcée. On peut citer dans ce domaine la demande WO2006/092271 qui décrit un procédé de production de polymères à partir d'acide acrylique préparé par voie enzymatique, notamment à partir de carbohydrate.

En ce qui concerne la transformation du glycérol par voie chimique, on peut citer la synthèse de l'acide acrylique en deux étapes à savoir l'obtention de l'acroléine par déshydratation de glycérol, qui est notamment décrite dans le brevet US 5,387,720, suivie d'une oxydation « classique » de l'acroléine pour obtenir l'acide acrylique.

FR 2 909 999 A décrit un procédé de préparation d'acide acrylique à partir d'une solution aqueuse de glycérol, comprenant une première étape de déshydratation du glycérol en acroléine, une étape intermédiaire consistant à condenser au moins en partie l'eau et les sous -produits lourds présents dans le flux issu de la première étape de déshydratation et une troisième étape d'oxydation de l'acroléine en acide acrylique. Les étapes de purification ne sont pas détaillées. US 2001/020111 A1 décrit un procédé de purification de l'acide acrylique, mais sans l'associer à une préparation de ce dernier à partir de glycérol.

La première étape de la fabrication d'acide acrylique à partir de glycérol conduit au même composé intermédiaire que le procédé de fabrication conventionnel à partir de propylène, à savoir l'acroléine selon la réaction :

CH₂OH-CHOH-CH₂OH → CH₂=CH-CHO + 2H₂O

qui est suivie de la deuxième étape d'oxydation selon la réaction

CH₂=CH-CHO + ½ O₂ → CH₂=CH-COOH

Les demandes de brevet EP 1.710.227, WO2006/136336 et WO2006/092272 décrivent de tels procédés de synthèse d'acide acrylique à partir de glycérol comportant l'étape de déshydratation en phase gaz en présence de catalyseurs constitués par des oxydes minéraux (mixtes ou non) à base d'aluminium, titane, zirconium, vanadium, etc., et l'étape d'oxydation en phase gaz de l'acroléine ainsi synthétisé en présence de catalyseurs à base d'oxydes de fer, molybdène, cuivre etc., seuls ou en combinaison sous forme d'oxydes mixtes.

L'acide acrylique est destiné à la mise en oeuvre par les industriels de procédés de polymérisation soit de l'acide acrylique, soit de ses dérivés esters, procédés qui sont conduits sous différentes formes en masse, en solution, en suspension, en émulsion. Ces procédés peuvent être très sensibles à la présence dans la charge de certaines impuretés, comme les aldéhydes ou les composés insaturés, qui peuvent parfois empêcher d'obtenir la valeur d'usage attendue, par exemple en limitant la conversion du monomère en polymère, en limitant la longueur de chaîne du polymère ou en interférant dans la polymérisation dans le cas de composés insaturés. D'autres impuretés, comme les composés saturés non polymérisables, peuvent être particulièrement gênants dans l'application finale, en modifiant les propriétés du produit fini, en conférant des propriétés toxiques ou corrosives au produit fini, ou encore en augmentant les rejets organiques polluants lors des étapes de fabrication du polymère et/ou du produit fini. Les exploitants se montrent exigeants en matière de spécifications de qualité de l'acide acrylique (ou de son ester). Celui-ci doit répondre à des seuils sévères en matière d'impuretés. En effet, les utilisateurs d'acide acrylique ou d'esters acryliques qui produisent des polymères mettent en oeuvre des recettes adaptées pour la production de leurs polymères à partir d'une qualité « standard » d'acide acrylique ou d'esters fabriquée aujourd'hui uniquement à partir de propylène. Une modification des recettes utilisées par ces utilisateurs, en vue des les adapter à une qualité différente d'acide acrylique ou d'esters produits par une autre voie que celle des procédés conventionnels ex-propylène présenterait des inconvénients importants pour ces sociétés utilisatrices. Hormis des coûts de recherche et développement supplémentaires, la production d'un type de polymères sur une même unité à partir de qualités différentes d'acide acrylique ou d'esters selon leur origine, fossiles ou bio-ressourcées (comme le glycérol), occasionnerait des coûts d'adaptation importants et une infrastructure de production plus compliquée.

La qualité de l'acide acrylique, c'est-à-dire sa teneur en différentes impuretés, jouant un grand rôle dans les processus de polymérisation ultérieurs, les industriels fabriquant cet acide acrylique ont été conduits à mettre enjeu toute une série d'étapes de purification afin d'obtenir cet acide acrylique « standard » qu'on appelle habituellement l'acide acrylique glacial (AAg). Cet AAg ne répond pas à des spécifications reconnues officiellement et ayant un caractère universel mais signifie pour chaque industriel le niveau de pureté à atteindre pour pouvoir conduire avec succès ses transformations ultérieures. A titre d'exemple, pour un acide acrylique ex-propylène, l'effluent de sortie de réacteur est soumis à une combinaison d'étapes qui peuvent différer par leur enchaînement selon le procédé : élimination des incondensables et de l'essentiel des composés très légers, en particulier l'acroléine intermédiaire de synthèse de l'acide acrylique (AA brut), déshydratation éliminant l'eau et le formol (AA déshydraté), élimination des légers (en particulier l'acide acétique), élimination des lourds, éventuellement élimination de certaines impuretés résiduelles par traitement chimique.

L'invention vise un procédé de fabrication d'un acide acrylique « standard » en utilisant le glycérol comme matière première qui sera transformé en deux étapes-déshydratation et oxydation - telles que mentionnées précédemment intégrées dans un processus global de purification.

Ce procédé présente avec le procédé de synthèse à partir du propylène une grande analogie dans la mesure où le produit intermédiaire, l'acroléine, issu de la première étape est le même et que la seconde étape est conduite dans les mêmes conditions opératoires. Cependant, la réaction de première étape du procédé de l'invention, réaction de déshydratation, est différente de la réaction d'oxydation du propylène du procédé habituel. La réaction de déshydratation menée en phase gaz est conduite au moyen de catalyseurs solides différents de ceux utilisés pour l'oxydation du propylène. L'effluent riche en acroléine issu de la première étape de déshydratation, destiné à alimenter la seconde étape d'oxydation de l'acroléine en acide acrylique, contient une quantité plus importante d'eau, et présente en outre des différences sensibles en matière de sous-produits découlant des mécanismes réactionnels mis en jeu se concrétisant par des sélectivités différentes dans chacune des deux voies.

Pour illustrer ces différences, le tableau 1 ci-dessous rassemble les données concernant la présence de divers acides dans l'acide acrylique brut, c'est-à-dire dans la phase liquide sortant du réacteur de seconde étape.

**Tableau 1**

| **Ratio massique impureté** / **AA (acide acrylique brut)** | Procédé ex-propylène | Procédé ex-glycérol |
|---|---|---|
| Acide acétique / AA | < 5% | > 10% |
| Acide propionique / AA | < 0.1% | > 0.5% |
| Acide 2-butènoïque/ AA | < 0.001% | > 0.01% |

Les ratio impuretés / AA dépendent des catalyseurs utilisés, de leur "âge" (dégradation des sélectivités au cours du temps) et des conditions opératoires. Dans le tableau 1 le ratio Acide 2-butènoïque / AA est mentionné < 0.001% pour le procédé ex-propylène; cependant bien que la demanderesse n'en ait jamais détecté dans l'AA ex-propylène, elle juge préférable d'écrire "< 10ppm" plutôt que 0% (résultat de ses analyses) afin de s'affranchir du problème de seuil de détection lié à la méthode d'analyse.

Le tableau 1 illustre certaines des principales différences, en terme de constituants de l'effluent liquide sortant du réacteur d'oxydation, entre les procédés ex-propylène et ex-glycérol. Naturellement, bien que cela ne soit pas mentionné dans le tableau, on trouve également dans l'acide acrylique brut, qu'il provienne du procédé ex-propylène ou du procédé ex-glycérol toute une série de composés oxygénés, alcools, aldéhydes, cétones, autres acides etc. dont la séparation, nécessaire, est connue de l'homme de l'art.

Les spécifications pour les qualités d'acide acrylique couramment utilisées pour la production de polymères d'acide acrylique et d'esters acryliques nécessitent de réduire les teneurs des impuretés du tableau 1 dans l'acide acrylique jusqu'aux valeurs figurant dans le tableau 2 ci-dessous.

**Tableau 2**

| **Concentration des impuretés dans l'AA (massique)** | Acide acrylique technique pour estérification | Acide acrylique glacial pour polymérisation |
|---|---|---|
| Acide acétique | < 0.2% | < 0.1% |
| Acide propionique | < 0.05% | < 0.05% |
| Acide 2-butènoïque | < 0.005% | < 0.001% |

L'acide acétique et l'acide propionique sont gênants en particulier parce qu'il ne sont pas transformés lors du processus de polymérisation, ils sont saturés et donc non polymérisables ; selon le procédé de polymérisation mis enjeu et les applications visées pour le polymère, ces impuretés peuvent rester dans le produit fini et risquer de conférer au produit fini des propriétés corrosives indésirables, ou se retrouver dans les rejets liquides ou gazeux générés par le procédé de polymérisation, et occasionner une pollution organique également non souhaitable.

L'acide 2-butènoïque, non synthétisé par le procédé ex-propylène mais présent sous ses 2 configurations (E, encore appelé acide crotonique, n° CAS : 107-93-7) et (Z, encore appelé acide isocrotonique, n° CAS : 503-64-0) dans le procédé ex-glycérol, est quant à lui particulièrement gênant car, du fait de sa double liaison, il est susceptible d'entrer en jeu dans le processus de polymérisation et donc de modifier les caractéristiques et la valeur d'usage du polymère final.

Pour atteindre les qualités d'acide acrylique citées dans le tableau 2, l'élimination de l'acide acétique peut être obtenue par distillation dans une fraction légère, opération généralement désignée par étêtage. Toutefois, la réduction de la concentration d'acide acétique dans le cadre du procédé ex-glycérol entraîne une perte conséquente d'acide acrylique dans la fraction légère, du fait d'une part de l'écart important existant entre sa teneur initiale dans l'acide acrylique brut et sa teneur visée dans l'acide acrylique technique pour estérification et d'autre part de l'existence de liaisons hydrogène existant entre les groupements carboxyliques des deux molécules. Cet inconvénient est important sur le plan économique, car l'obtention d'un acide acrylique glacial à teneur en acide acétique inférieure à 0.1 % poids ne peut se faire qu'au détriment du taux de récupération de l'acide acrylique sortant du réacteur d'oxydation.

La réduction de la teneur en acide 2-butènoïque par voie de distillation doit s'obtenir par passage dans une fraction lourde, cette dernière séparation s'avérant cependant difficile.

Pour ce qui concerne l'acide propionique, la différence de volatilité extrêmement faible existant entre cette impureté à éliminer et l'acide acrylique à purifier (de l'ordre de 1°C) empêche toute purification dans des conditions économiquement acceptables par distillation.

La présente invention a pour but de produire à partir de glycérol un acide acrylique répondant à tous les critères de qualité des monomères habituellement utilisés comme matière première dans les procédés de polymérisation d'acide acrylique et esters, par un procédé économique.

L'invention a pour objet un procédé de fabrication d'acide acrylique bio-ressourcé à partir de glycérol comportant les étapes suivantes :
- déshydratation catalytique en phase gaz du glycérol en acroléine, (1)
- condensation partielle par refroidissement et extraction d'une partie de l'eau contenue dans le milieu réactionnel de (1), (1')
- oxydation catalytique en phase gaz de l'acroléine en acide acrylique, (2)
- extraction de l'acide acrylique contenu dans l'effluent de l'oxydation par absorption à l'eau avec refroidissement et élimination de la fraction légère riche en composés gazeux très légers non condensables, (3)
- séchage de la solution d'acide acrylique par distillation en présence d'un solvant non miscible à l'eau, (4)
- distillation de la solution ainsi obtenue pour éliminer les composés légers (étêtage), (5)
- distillation de la fraction lourde issue de l'étape précédente (5) pour éliminer les composés lourds (équeutage), (6)
combinées avec une étape finale d'extraction de l'acide acrylique par cristallisation fractionnée appliquée à l'un des effluents suivants : la fraction lourde de (4), la fraction lourde de (5) ou la fraction légère de (6).

Pour la mise en oeuvre du procédé on utilise généralement un flux alimentant le réacteur de l'étape (1) contenant le glycérol et de l'eau, avec un ratio massique eau / glycérol pouvant varier dans de larges mesures, par exemple entre 0,04 / 1 et 9 / 1 et de préférence entre 0,7 / 1 et 3 / 1. La réaction de déshydratation, étape (1), qui est une réaction équilibrée mais favorisée par un niveau de température élevée, est effectuée en général en phase gaz dans le réacteur en présence d'un catalyseur à une température allant de 150°C à 500°C, de préférence comprise entre 250°C et 350°C, et une pression absolue comprise entre 1 et 5 bars (100 et 500 kPa). On peut aussi l'effectuer en présence d'oxygène ou d'un gaz contenant de l'oxygène comme décrit dans les demandes WO 06/087083 et WO 06/114506.

La réaction de déshydratation du glycérol est généralement effectuée, sur des catalyseurs solides acides. Les catalyseurs qui conviennent sont des matériaux utilisés dans un milieu réactionnel gazeux ou liquide, en phase hétérogène, qui ont une acidité de Hammett, notée H₀ inférieure à +2. Comme indiqué dans le brevet US 5,387,720 qui fait référence à l'article de K. Tanabe et al dans "Studies in Surface Science and Catalysis", Vol 51, 1989, chap 1 et 2, l'acidité de Hammett est déterminée par titration amine à l'aide d'indicateurs ou par adsorption d'une base en phase gazeuse.

Ces catalyseurs peuvent être choisis parmi des matériaux siliceux naturels ou de synthèse ou les zéolithes acides ; des supports minéraux, tels que des oxydes, recouverts par des acides inorganiques, mono, di, tri ou polyacides ; des oxydes ou oxydes mixtes ou encore des hétéropolyacides ou sels d'hétéropolyacides.

Ces catalyseurs pourront généralement être constitués par un sel d'hétéropolyacide dans lequel des protons dudit hétéropolyacide sont échangés avec au moins un cation choisi parmi les éléments appartenant aux Groupes I à XVI de la Classification Périodique des Eléments, ces sels d'hétéropolyacide contenant au moins un élément choisi parmi le groupe comprenant W, Mo et V.

Parmi les oxydes mixtes, on peut citer particulièrement ceux à base de fer et de phosphore et ceux à base de césium, phosphore et tungstène.

Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale Ta₂O₅, oxyde de niobium Nb₂O₅, alumine Al₂O₃, oxyde de titane TiO₂, zircone ZrO₂, oxyde d'étain SnO₂, silice SiO₂ ou silico-aluminate SiO₂-Al₂O₃, imprégnés de fonctions acides telles que borate BO₃, sulfate SO₄, tungstate WO₃, phosphate PO₄, silicate SiO₂, ou molybdate MoO₃, ou un mélange de ces composés.

Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate, les alumines ou silices phosphatées, les hétéropolyacides ou sels d'hétéropolyacides, les phosphates de fer et les phosphates de fer comprenant un promoteur.

Le milieu réactionnel sortant du réacteur de déshydratation a une teneur en eau importante due à la charge de glycérol (solution aqueuse) et à la réaction elle-même. Une étape supplémentaire (1') de condensation partielle de l'eau telle que par exemple celle décrite dans la demande de brevet WO 08/087315 au nom de la déposante, permettra d'en éliminer une partie, de façon à amener ce gaz à une composition sensiblement identique à celle du procédé ex-propylène, pour alimenter la deuxième étape d'oxydation de l'acroléine en acide acrylique. Par composition sensiblement identique, on entend en particulier des concentrations en acroléine, eau et oxygène proches. Cette étape de condensation (1') doit être conduite avec un refroidissement à une température permettant d'obtenir, après élimination de la phase condensée, un flux gazeux contenant de l'eau et de l'acroléine dans un ratio molaire eau / acroléine de 1,5 / 1 à 7 / 1 Cette condensation partielle de l'eau permet d'éviter une dégradation du catalyseur de 2^{éme} étage d'oxydation de l'acroléine en acide acrylique et d'éviter lors des étapes ultérieures l'élimination de grandes quantités d'eau qui est coûteuse et qui risque d'entraîner des pertes en acide acrylique. En outre elle permet d'éliminer une partie des impuretés « lourdes » formées lors de la déshydratation

La réaction d'oxydation, étape (2) s'effectue en présence d'oxygène moléculaire ou d'un mélange contenant de l'oxygène moléculaire, à une température allant de 200°C à 350°C, de préférence de 250°C à 320°C, et sous une pression allant de 1 à 5 bars en présence d'un catalyseur d'oxydation.

Comme catalyseur d'oxydation, on utilise tous types de catalyseurs bien connus de l'homme de l'art pour cette réaction. Généralement sont utilisés des solides contenant au moins un élément choisi dans la liste Mo, V, W, Re, Cr, Mn, Fe, Co, Ni, Cu, Zn, Sn, Te, Sb, Bi, Pt, Pd, Ru, Rh, présent sous la forme métallique ou sous forme d'oxyde, de sulfate ou de phosphate. En particulier, sont utilisées les formulations contenant Mo et/ou V et/ou W et/ou Cu et/ou Sb et/ou Fe comme constituants principaux.

Le mélange gazeux issu de l'étape (2) est constitué, en dehors de l'acide acrylique :
- de composés légers incondensables dans les conditions de températures et de pression habituellement mises en oeuvre : azote, oxygène non converti, monoxyde et dioxyde de carbone formés en faible quantité par oxydation ultime,
- de composés légers condensables : en particulier l'eau, générée par la réaction de déshydratation ou présente comme diluant, l'acroléine non convertie, des aldéhydes légers, comme le formaldéhyde et l'acétaldéhyde, l'acide formique, l'acide acétique et l'acide propionique
- de composés lourds : furfuraldéhyde, benzaldéhyde, acide et anhydride maléique, acide 2-butènoïque, acide benzoïque, phénol, protoanémonine.

L'étape (3) consiste en une extraction de l'acide acrylique par une absorption à contre-courant avec de l'eau. Pour cela, on introduit le gaz issu du réacteur en pied d'une colonne d'absorption où il rencontre à contre-courant de l'eau introduite en tête de colonne. Les composés légers (principalement acétaldéhyde et acroléine) sont pour l'essentiel éliminés en tête de cette colonne d'absorption. L'eau utilisée comme solvant absorbant peut être amenée par une source extérieure au procédé, mais sera constituée préférentiellement pour partie ou totalement par récupération à partir d'au moins un des flux gazeux réactionnels issus des étapes réactionnelles initiales par exemple l'eau issue des étapes (1') et (4), à savoir l'eau condensée dans l'étape 1', ou l'eau récupérée à partir du flux de tête de colonne de séchage azéotropique. Les conditions opérationnelles de cette étape d'absorption sont les suivantes :
Le mélange réactionnel gazeux est introduit en pied de colonne à une température comprise entre 130°C et 250°C. L'eau est introduite en tête de colonne à une température comprise entre 10°C et 60°C. Les quantités respectives d'eau et de mélange réactionnel gazeux sont telles que le ratio massique eau/ acide acrylique est compris entre 1/1et 1/4. L'opération est conduite à la pression atmosphérique.

Dans une variante préférée de mise en oeuvre du procédé, lors d'une étape (3') on procède à la récupération de l'acroléine, contenue dans la fraction liquide issue de (3), par distillation ou stripping avec un gaz. Dans cette variante du procédé, la colonne d'absorption peut être couplée avec une colonne de distillation des composés très légers, essentiellement l'acroléine non convertie à l'issue de la réaction, présente en faible concentration dans la solution aqueuse d'acide acrylique récupérée en pied de colonne d'absorption. Cette colonne de distillation, fonctionnant sous une pression de 6.10³ à 7.10⁴ Pa, est alimentée en tête par le flux de pied de colonne d'absorption précédente, et permet d'éliminer en tête un flux d'acide acrylique enrichi en acroléine, qui est recyclé en partie inférieure de la colonne d'absorption (3), pour une élimination finale en tête de cette même colonne. On obtient ainsi un mélange aqueux d'acide acrylique dans l'eau (ratio massique 1/1 à 4/1) débarrassé de l'essentiel de l'acroléine non convertie, que l'on nomme "acide acrylique brut". La récupération de l'acroléine peut également être réalisée par stripping avec un gaz tel que l'air ou un mélange de gaz inerte contenant préférentiellement de l'oxygène

Cette étape est facultative mais en son absence l'acide acrylique brut sera plus concentré en acroléine qui devra être éliminée lors de l'étape ultérieure d'étêtage. Par ailleurs, cette étape (3') permet de récupérer et recycler l'acroléine à la section réaction (2) et ainsi d'augmenter le rendement global du procédé.

L'étape (4) est une étape de déshydratation ou séchage qui est réalisée en présence d'un solvant de l'acide acrylique non miscible à l'eau. Cette étape de déshydratation peut être réalisée par extraction liquide - liquide de l'acide acrylique en présence du solvant, suivie par une étape de séparation du monomère, acide acrylique, par distillation.

Cette phase de déshydratation est décrite dans de nombreux brevets, voir par exemple le brevet FR 2.119.764, avec la méthylisobutylcétone (MIBK) comme solvant, ou le brevet US 3,689,541, avec la triméthylcyclohexanone comme solvant, ou par distillation en présence de solvant ou de mélanges de solvants formant un azéotrope hétérogène avec l'eau, comme les acétates ou la méthylisobutylcétone, tel que décrite par exemple dans le brevet FR 2.554.809 ou encore des solvants formant en outre un mélange azéotropique avec l'acide acétique comme le toluène, tel que décrit par exemple dans le brevet JP 03.181.440.

Dans le procédé de l'invention, on utilisera de préférence pour cette étape de déshydratation une distillation azéotropique à l'aide d'un solvant tel que MIBK. La colonne de distillation qui fonctionne sous une pression de 6.10³ à 7.10⁴ Pa, est équipée d'un décanteur qui reçoit le flux de tête de colonne après condensation et assure la séparation d'une phase organique supérieure essentiellement constituée de MIBK, totalement recyclée en reflux en tête de colonne, et d'une phase aqueuse contenant l'eau et la majeure partie du formaldéhyde. La puissance de chauffe imposée au bouilleur de la colonne est régulée de façon à obtenir un débit de reflux de solvant tel que le ratio massique de solvant renvoyé en reflux et de l'eau contenue dans l'acide acrylique brut alimentant la colonne corresponde au mélange azéotropique théorique. Le flux obtenu en pied de colonne, l'acide acrylique déshydraté, est essentiellement exempt d'eau (généralement moins de 1% poids).

Dans une variante de réalisation, cette colonne peut être couplée à une deuxième colonne de récupération du solvant, de façon à récupérer dans le flux aqueux décanté en tête de colonne de distillation azéotropique les traces de solvant solubilisées dans la phase aqueuse. Ces faibles quantités de solvant distillées et condensées en tête de cette colonne de récupération de solvant, fonctionnant sous pression atmosphérique, sont ensuite recyclées dans le décanteur de la colonne précédente. Le flux aqueux de pied de cette colonne de récupération de solvant est éliminé.

L'étape (5) est une étape d'élimination des composés légers, en particulier l'acide acétique et l'acide formique, par distillation ; elle est généralement dénommée « étêtage ». Le flux d'acide acrylique déshydraté obtenu en pied de la colonne de distillation azéotropique est envoyé en partie médiane d'une colonne à distiller qui fonctionne sous une pression de tête de l'ordre de 2.10³ à 2.10⁴ Pa. Le flux de pied de colonne contient l'acide acrylique débarrassé de l'essentiel des composés légers. Le flux de tête de colonne, riche en acide acétique et acide formique, peut éventuellement être traité de façon complémentaire pour récupérer dans une deuxième colonne en série les faibles quantités d'acide acrylique entraînées avec le flux de tête de colonne.

L'étape (6) est une étape de séparation des composés lourds par distillation. Le flux de pied de la colonne précédente d'étêtage est introduit en pied d'une colonne de distillation fonctionnant sous une pression de tête de l'ordre de 2.10³ à 2.10⁴ Pa. On obtient en tête un flux d'acide acrylique purifié, dit de grade technique.

Les différentes étapes de séparation par distillation imposent en raison des conditions thermodynamiques mises en oeuvre d'ajouter aux flux traités des inhibiteurs de polymérisation afin d'éviter la formation de composés lourds formés par polymérisation de l'acide acrylique, qui sont préjudiciables au bon fonctionnement de l'ensemble. Les inhibiteurs de polymérisation généralement utilisés pour les étapes de purification de l'acide acrylique sont les produits phénoliques, comme l'hydroquinone ou l'éther méthylique de l'hydroquinone, les dérivés de la phénothiazine, des composés de la famille des thiocarbamates, comme le di n-butyl dithiocarbamate de cuivre, des dérivés aminés, comme les hydroxylamines, l'hydroxydiphénylamine, ou de la famille des phénylène diamines, des dérivés nitroxydes de la 4-hydroxy 2,2,6,6-tetraméthyl-1-oxyl-pipéridine (TEMPO), comme le 4-hydroxy-TEMPO ou le 4-oxo-TEMPO, ou encore des sels métalliques, comme l'acétate de manganèse. Ces inhibiteurs peuvent être utilisés seuls ou en combinaison, et sont en outre préférentiellement introduits en association avec un gaz contenant de l'oxygène.

Ces inhibiteurs de polymérisation sont généralement des composés lourds, dont la volatilité est plus faible que celle de l'acide acrylique. Ils sont éliminés en fond des colonnes. En revanche, leur concentration dans la phase vapeur à l'intérieur des colonnes de distillation est faible et insuffisante pour éviter l'initiation de polymères. Pour éviter l'apparition et l'accumulation de polymères, ces additifs sont habituellement introduits dans les flux liquides alimentant les équipements, mais aussi en tête et en différents points des colonnes et équipements, de façon à assurer un reflux constant et homogène de solution riche en inhibiteurs de polymérisation sur toutes les parties des équipements. En général, ils sont envoyés en solution dans un liquide, par exemple dans l'acide acrylique ou dans l'eau si l'étape de purification concerne des flux aqueux.

Dans le procédé de l'invention la dernière étape du processus de purification de l'acide acrylique bio-ressourcée est une séparation par cristallisation fractionnée ainsi associée aux étapes précédentes de purification.

La cristallisation fractionnée est une technique de séparation bien connue. Elle peut être mise en oeuvre sous différentes formes, cristallisation dynamique, cristallisation statique ou cristallisation en suspension. On peut citer à ce sujet le brevet français 77 04510 du 17/02/1977 (BASF) et les brevets US 5,504,247 (Sulzer) et 5,831,124 (BASF) et 6,482,981 (Nippon Shokubai) dont certains visent la purification de l'acide acrylique synthétisé par oxydation du propylène.

La technique la plus utilisée est la cristallisation fractionnée en film tombant, cristallisation dynamique, éventuellement associée à une cristallisation statique en milieu fondu.

Le cristallisation à film tombant est généralement opérée dans un échangeur tubulaire, multitubulaire en pratique, chaque tube étant alimenté en continu (en tête), par :
- un flux liquide (solution ou fondu), du composé à purifier, l'acide acrylique (AA) dans le procédé, tombant en film de préférence le long de la paroi interne du tube, réceptionné en pied de tube et recyclé en tête (boucle fermée) pendant le temps nécessaire à la cristallisation de la quantité de composé (AA) décidée par l'opérateur,
- un flux de fluide caloporteur, par exemple éthylèneglycol - eau ou méthanol - eau, tombant en film, de préférence le long de la paroi externe du tube, également recirculé tout au long de la cristallisation au sein du tube et qui apportera le froid ou la chaleur nécessaire à l'opération des étapes de chacun des étages.

Le procédé est une combinaison d'étages successifs, qui comprennent chacun 3 étapes :
- cristallisation : la température du fluide caloporteur est abaissée selon un gradient de température négatif à partir d'une température légèrement supérieure à la température de cristallisation de l'acide acrylique dans le milieu, de l'ordre de 14 °C. Des cristaux se forment en couche de plus en plus épaisse à la surface des tubes. Lorsque environ 30 à 80% d'AA mis en circulation est cristallisé, après égouttage, on transfert la fraction liquide restante (eaux-mères riches en impuretés) dans un récepteur.
- ressuage : la température du fluide caloporteur est réchauffée selon un gradient de température positif pour éliminer par fusion les impuretés emprisonnées sous forme d'inclusions dans la couche de cristaux d'acide acrylique en formation ; celles-ci sont principalement localisées au niveau de la couche la plus externe qui est en contact avec le flux recirculé de plus en plus riche en impuretés. Lors du ressuage, les premières molécules à fondre sont des mélanges eutectiques d'impuretés et d'AA, les impuretés localisées au sein de la couche de cristaux migrent vers la couche externe, c'est-à-dire celle qui était en contact avec le flux recirculé. Une faible partie de cette couche de cristal est ainsi fondue et transférée dans un récepteur, de préférence le même récepteur que celui des eaux-mères récupérées lors de l'étape de cristallisation. A cette étape de ressuage, on peut substituer une technique de lavage qui consiste à éliminer les impuretés présentes à la surface par lavage avec de l'AA pur, introduit préférentiellement à température légèrement plus élevée que la température de fusion de la couche d'AA. Cette technique est cependant à priori moins efficace.
- fusion : la température du fluide caloporteur est rapidement augmentée au-delà du point de fusion de l'AA (14°C) et doit de préférence rester inférieure à une température maximum au delà de laquelle on peut craindre une polymérisation (explosive) du milieu : cette température maximum est de l'ordre de 35-40°C pour rester en sécurité pour faire fondre la couche de cristaux d'AA purifié. Le liquide purifié récupéré est placé dans un deuxième récepteur.

A partir du flux à purifier, l'ensemble des 3 étapes décrites représente un premier étage de purification. Le liquide purifié à l'issue de ce premier étage peut à nouveau subir une succession des 3 étapes décrites dans un 2ème étage de purification (phase de purification). Les eaux-mères issues de ce 2ème étage sont plus pures que celles de l'étage précédent et peuvent donc être utilisées en mélange avec une nouvelle charge d'AA à purifier dans l'étage n° 1. La même opération peut être réalisée dans un troisième étage de purification, les eaux-mères de ce troisième étage pouvant être recyclées dans la charge du 2^{éme} étage, le produit pur étant récupéré par fusion des cristaux. D'une manière générale, les eaux-mères de l'étage de purification "n" peuvent être recyclées en les mélangeant avec le flux d'alimentation de l'étage de purification "n-1"

Lors des phases de purification, les inhibiteurs de polymérisation contenus dans les mélanges à purifier sont traités comme des impuretés et sont donc éliminés dans les eaux-mères. Pour éviter la formation de polymère dans le cristallisat fondu, on ajoute préférentiellement un inhibiteur compatible en nature et concentration avec l'usage final du monomère. Cet ajout sera en particulier mis en oeuvre lors de la dernière étape de fusion d'un étage alimenté par un flux exempt d'inhibiteur de polymérisation, comme par exemple le dernier étage de purification "n" alimenté uniquement avec un flux purifié de l'étage "n-1".

Les eaux-mères collectées à la suite du premier étage de purification peuvent être traitées dans un étage "-1" selon le même processus à trois étapes. Le cristallisat récupéré peut être utilisé comme complément de charge d'alimentation du premier étage. Les eaux-mères de l'étage "-1" sont alors traitées selon le même processus pour une nouvelle séparation dont le cristallisat entrera comme charge de l'étage juste supérieur et les eaux-mères soumises à nouveau au processus dans un étage inférieur "-2". Les étages "-1", "-2", etc. constituent les étages de concentration (les étages successifs permettent de concentrer les impuretés dans les flux d'eaux-mères). De manière générale, les eaux-mères des étages de concentration "n" sont traitées selon le même processus à 3 étapes dans l'étage postérieur "n-1". La répétition de ces opérations (phase de concentration) permettra de concentrer les impuretés dans un flux d'eaux-mères de plus en plus riche en impuretés tandis que les fractions d'acide acrylique pur seront remontées vers l'étage initial. Ainsi, on peut récupérer l'acide acrylique entraîné dans les eaux-mères initiales pour améliorer le rendement de récupération et par ailleurs obtenir un mélange « enrichi » en impuretés.

Les étages de concentration successifs sont caractérisés par des flux d'eaux-mères de plus en plus concentrées en impuretés, au fur et à mesure qu'on accumule ces étages. Ce faisant, la température de cristallisation de ces mélanges est de plus en plus basse, ce qui a pour effet d'augmenter le coût énergétique de refroidissement. Par ailleurs, le temps nécessaire pour cristalliser une même quantité d'acide acrylique est de plus en plus long, ce qui a pour conséquence de diminuer la productivité de la purification pour une même surface de cristallisation. Par conséquent, on arrêtera en général de préférence le nombre des étapes de concentration avant que la concentration totale des impuretés dans les eaux-mères ne dépasse 50% poids du flux.

Selon la pureté du produit de départ, du produit purifié attendu et le rendement de récupération d'AA souhaité, le procédé complet pour une qualité initiale d'AA de type « technique » comprend généralement entre 1 et 4 étages de purification et entre 1 et 4 étages pour la concentration des impuretés.

Pour améliorer encore le rendement de récupération, on peut aussi réaliser le dernier étage de concentration dans un cristalliseur statique. Dans ce cas, le mélange à cristalliser est placé en contact avec une paroi refroidie. Il peut s'agir par exemple d'un échangeur constitué de plaques métalliques circulées par un fluide caloporteur, plongées dans une cuve contenant les eaux-mères de cristallisation des étages précédents. L'AA forme une couche de cristal sur la paroi des plaques, puis on élimine les eaux-mères et la couche cristallisée est fondue pour être traitée ensuite dans un étage supérieur de cristallisation dynamique en film tombant.

Parmi les problèmes posés par le traitement de la phase liquide de l'effluent issu du réacteur d'oxydation est la présence d'une quantité très importante d'acide acétique dont la séparation au niveau de l'étape d'étêtage (étape 5) est délicate ainsi que cela a été indiqué précédemment et qui implique l'utilisation d'une colonne comportant un nombre de plateaux importants entraînant une différence de pression et de température importantes entre le fond et la tête de colonne. Dans le cas de la mise en oeuvre d'une étape de purification par cristallisation en aval de cette étape d'étêtage, les conditions opératoires de cette dernière pour éliminer l'acide acétique pourront être simplifiées en visant un taux d'élimination moins drastique. En effet, l'acide acétique résiduel à l'issue de l'étape d'étêtage, pourra être éliminé lors de l'étape postérieure de cristallisation. Le gain qui pourra être induit par l'étape de cristallisation sur l'étape d'étêtage est donc une diminution du nombre d'étages théoriques de la colonne de distillation (donc de la hauteur de la colonne) et par conséquent un gain d'investissement. Un deuxième avantage est une diminution de la température en pied de colonne, imposée par la perte de charge dans la colonne, elle-même proportionnelle au nombre de plateaux, et par conséquent une diminution du risque de formation de composés lourds du type dimère d'acide acrylique (acide β-acryloxypropionique) et de polymères.

La localisation de l'étape, finale, de cristallisation dans la chaîne de purification de l'acide acrylique dépendra de la charge initiale traitée, la nature et la source du glycérol utilisé dans le procédé, des spécifications de pureté à atteindre pour l'acide acrylique final et enfin de critères économiques liés aux économies de coûts au niveau des distillations comparés à un surcoût dû à une augmentation du nombre d'étages de cristallisation.

L'application du traitement par cristallisation fractionnée à la fraction légère de l'étape (6) présente l'avantage de réaliser pleinement les objectifs recherchés dans la présente demande avec un rendement de récupération de l'acide acrylique particulièrement performant dans la dernière étape de cristallisation (supérieur à 97%) et un rendement global de purification meilleur que celui du procédé classique par distillation avec un degré de pureté de l'acide acrylique au moins aussi bon.

L'application du traitement par cristallisation fractionnée à la fraction lourde de l'étape (4) permet de réduire les coûts d'investissement de l'unité en réduisant le nombre de colonnes de purification ainsi que les coûts matière, comme les inhibiteurs de polymérisation. En outre, on diminue le risque d'encrassements par polymérisation. Le rendement de récupération de la dernière étape de cristallisation (>90%) n'est pas aussi bon que dans le cas précédent, mais le rendement global de récupération reste meilleur que celui du procédé classique par distillation.

L'application du traitement par cristallisation fractionnée à la fraction lourde de l'étape (5) est intermédiaire entre les 2 premières, avec un rendement de récupération global qui reste supérieur à celui du procédé classique par distillation.

L' acide acrylique bio-ressourcé obtenu selon le procédé de l'invention peut être utilisé pour la fabrication d'homopolymères et copolymères produits par polymérisation de l'acide acrylique et éventuellement d'autres monomères insaturés, par exemple la fabrication de polymères superabsorbants obtenus par polymérisation dudit acide partiellement neutralisé, ou la polymérisation dudit acide suivie d'une neutralisation partielle de l'acide polyacrylique obtenu.

L' acide acrylique bio-ressourcé peut aussi être utilisé pour la fabrication de polymères ou de copolymères par polymérisation des dérivés dudit acide sous forme ester ou amide.

Le procédé de fabrication de l'acide acrylique selon l'invention est illustré par les exemples suivants.

### Exemple 1 : Fabrication d'acide acrylique brut à partir de glycérol

L'étape préliminaire consiste à purifier le glycérol brut obtenu à partir d'huile végétale, en éliminant les sels. La solution de glycérol brut est constituée en poids de 89,7% de glycérol, 3,9% d'eau, 5,1% de chlorure de sodium. Ce flux (6 400g) est envoyé en continu en alimentation d'un réacteur agité de 2 litres chauffé par un chauffe réacteur électrique extérieur. Les vapeurs de glycérol et d'eau sont condensées dans un réfrigérant et récupérées dans un récepteur. Cette opération de purification est réalisée sous pression de 670 Pa (5mm Hg). On obtient 5 710g d'une solution de glycérol exempte de chlorure de sodium.

Passant à l'étape (1) du procédé, on réalise la réaction de déshydratation du glycérol en acroléine et la condensation (1') d'une partie de l'eau. La réaction de déshydratation est effectuée en phase gaz dans un réacteur en lit fixe en présence d'un catalyseur solide constitué par une zircone tungstée ZrO₂-WO₃ à une température de 320°C, sous pression atmosphérique. Un mélange de glycérol (20% massique) et eau (80% massique) est envoyé dans un vaporiseur, en présence d'air, dans un ratio molaire O₂ / glycérol de 0,6 / 1. Le milieu gazeux sortant du vaporiseur à 290°C est introduit dans le réacteur, constitué d'un tube de diamètre 30mm chargé de 390ml de catalyseur, plongé dans un bain de sel (mélange eutectique KNO₃, NaNO₃, NaNO₂) maintenu à la température de 320°C.

En sortie du réacteur, le mélange réactionnel gazeux est envoyé en pied d'une colonne de condensation. Cette colonne est constituée d'une section inférieure remplie d'anneaux raschig, surmontée d'un condenseur circulé par un fluide caloporteur froid. La température de refroidissement dans les échangeurs est adaptée de façon à obtenir en tête de colonne une température des vapeurs de 72°C sous pression atmosphérique. Dans ces conditions, la perte d'acroléine en pied de colonne de condensation est inférieure à 5%.

Dans l'étape suivante (2), le mélange gazeux est introduit, après addition d'air (ratio molaire O₂ / acroléine de 0,8 / 1) et d'azote en quantité nécessaire pour obtenir une concentration d'acroléine de 6,5% molaire, en alimentation du réacteur d'oxydation de l'acroléine en acide acrylique. Ce réacteur d'oxydation est constitué d'un tube de diamètre 30mm chargé de 480ml d'un catalyseur commercial d'oxydation d'acroléine en acide acrylique à base d'oxydes mixtes d'aluminium, molybdène, silicium, vanadium et cuivre plongé dans un bain de sel identique à celui décrit ci-dessus, maintenu lui à une température de 345°C. Avant introduction sur le lit catalytique, le mélange gazeux est préchauffé dans un tube plongeant également dans le bain de sel.

En sortie du réacteur d'oxydation, le mélange gazeux est introduit en pied d'une colonne d'absorption, étape (3), fonctionnant sous pression atmosphérique. Cette colonne est remplie de garnissage vrac en inox du type ProPak. En partie inférieure, sur 1/3 de sa hauteur totale, la colonne est équipée d'une section de condensation, en tête de laquelle on recycle une partie du mélange condensé récupéré en pied de colonne, après refroidissement dans un échangeur externe. La partie supérieure de la colonne est refroidie par échange thermique à travers la paroi. La température des vapeurs en tête de colonne est de 25°C, celle de la solution aqueuse d'acide acrylique brut obtenue en pied de colonne est de 35°C. Le produit obtenu en pied (acide acrylique brut) contient 40% d'eau et un mélange d'acide acrylique (produit majoritaire) et d'impuretés, présentes dans des ratios massiques "impuretés / AA" indiqués dans le tableau 3 ci-dessous. On introduit en continu, dans la boucle de recirculation en pied de colonne, une solution aqueuse d'hydroquinone (HQ), à concentration de 0,1% poids par rapport à l'acide acrylique.

### Ex.emple 2 (comparatif) : Fabrication d'acide acrylique brut à partir de propylène

Le réacteur d'oxydation du propylène en acide acrylique est constitué de 2 tubes réactionnels en série, plongés dans des compartiments indépendants contenant des bains de sels fondus chauffés (mélange eutectique de 53% KNO₃, 40% NaNO₂ et 7% NaNO₃). Les 2 tubes réactionnels ont une hauteur de 1m, un diamètre de 25mm et sont remplis respectivement d'un catalyseur commercial d'oxydation du propylène en acroléine catalyseur à base d'oxydes de molybdène, bismuth, fer et silicium et de catalyseur d'oxydation de l'acroléine en acide acrylique identique à celui utilisé dans l'exemple 1. Le bain de sel du compartiment contenant le premier tube de réaction est chauffé à une température de 362°C, celui du 2^{éme} étage de réaction à une température de 345°C . Le premier tube de réaction est alimenté à un débit de 546 Nl/h par un mélange constitué en volume de 7% de propylène, 7% d'eau, 26% d'azote, 60% d'air. Le gaz réactionnel sortant du tube est envoyé dans le deuxième tube réactionnel.

Le flux réactionnel sortant du deuxième réacteur est envoyé vers le pied d'une colonne d'absorption identique à celle de l'exemple 1, dans les mêmes conditions que cet exemple 1.

Le produit obtenu en pied (acide acrylique brut) contient 35% d'eau et un mélange d'acide acrylique (produit majoritaire) et d'impuretés, présentes dans des ratios massiques "impuretés / AA" indiqués dans le tableau 3 ci-dessous.

**Tableau 3**

| ***Rapports massiques "impuretés* / *Acide acrylique"*** | **Exemple 1** | **Exemple 2** |
|---|---|---|
| Formaldéhyde | 0.38% | 1.37% |
| Acide acétique | 14.44% | 3.27% |
| Acide propionique | 0.81% | 0.05% |
| Furfural | 0.01% | 0.02% |
| Protoanémonine | 0.02% | 0.02% |
| Benzaldéhyde | 0.03% | 0.03% |
| Acide trans-2-butènoïque (crotonique) | 0.04% | 0.00% |
| Acide Maléique | 0.41% | 0.67% |

### Exemple 3 : Purification de l'AA brut obtenu ex-glycérol en AA technique

La solution aqueuse obtenue est soumise à une étape (4) de séchage par une distillation pour éliminer l'eau sous forme d'un mélange azéotropique avec la méthylisobutylcétone (MIBK). La colonne, garnie d'éléments ProPak représentant une efficacité de 15 plateaux théoriques, est alimentée en son milieu par l'AA brut, et en tête par la MIBK, dans un ratio massique MIBK / eau contenue dans l'AA brut de 3 / 1. Une solution de stabilisants dans la MIBK est injectée en continu en tête de colonne, contenant les stabilisants hydroquinone, phénothiazine et dibutyldithiocarbamate de butyle (respectivement : 35ppm, 70ppm, 35ppm par rapport à l'acide acrylique contenu dans le flux d'alimentation). Le mélange azéotropique distille à une température de tête de 45°C sous une pression de 1,2.10⁴ Pa.

L'acide acrylique déshydraté récupéré en pied de colonne ne contient plus que 0.4% d'eau.

Il est envoyé, étape (5), en alimentation d'une colonne d'étêtage, qui permet d'éliminer en tête, les composés légers, essentiellement l'acide acétique. Cette colonne, garnie d'éléments ProPak (20 plateaux théoriques), est alimentée en son milieu par le flux d'AA déshydraté, et on distille en tête un flux riche en acide acétique sous pression de 1,3.10⁴ Pa, à une température de tête de 77°C, avec un taux de reflux de 7 / 1. En tête de colonne de distillation, on introduit une solution de stabilisants dans l'acide acrylique technique contenant les stabilisants hydroquinone et dibutyldithiocarbamate de butyle (400 ppm par rapport à l'acide acrylique contenu dans le flux d'alimentation). Le rendement de récupération de l'acide acrylique dans cette étape est de 97%.

L'acide acrylique étêté récupéré en pied de cette colonne titre 0.07% d'acide acétique. Il est envoyé, étape (6) en alimentation d'une colonne d'équeutage garnie de 17 plateaux perforés à déversoirs, qui permet d'éliminer les composés lourds en pied. Cette colonne fonctionne sous une pression de 6,7.10³ Pa, avec une température de tête de 73°C, avec un taux de reflux de 0,5/1. Au niveau du plateau supérieur de la colonne de distillation, on introduit une solution de stabilisants dans l'acide acrylique technique contenant les stabilisants phénotiazine et dibutyldithiocarbamate de butyle (400ppm par rapport à l'acide acrylique contenu dans le flux d'alimentation) et le flux de distillat condensé est additionné d'une solution d'hydroquinone dans l'AA pur (200ppm par rapport à l'acide acrylique distillé). L'acide acrylique obtenu en tête de colonne constitue l'acide acrylique technique (AAT).

Les analyses de l'acide acrylique de grade technique montrent que le produit contient 0.07% d'acide acétique, 0.66% d'acide propionique, 0.11% d'anhydride maléique, 0.11% d'eau, 0.023% d'acide 2-butènoïque, 0.01% de furfural, 0.02% de benzaldéhyde, 0.01% de protoanémonine et 0.02% d'acroléine.

Le rendement de récupération de l'acide acrylique dans cette étape est de 95,5%.

### Exemple 4 : Purification de l'AA technique ex-glycérol par cristallisation

Le flux d'acide acrylique de qualité technique obtenu dans l'exemple 3 est soumis à une série d'étages de purification et de concentration par cristallisation fractionnée, telles que décrits dans la présente demande. Le montage utilisé est un cristalliseur à flux tombant constitué d'un tube vertical en inox rempli de fluide caloporteur (mélange éthylèneglycol - eau) circulant en circuit fermé, via une pompe, à travers un échangeur de chaleur externe programmable en rampe de température (bain cryostatique Lauda). Ce tube est alimenté en tête sous forme d'un film liquide coulant uniformément sur sa paroi externe. Le liquide constitué du mélange à cristalliser, récupéré dans un bac récepteur en pied, recircule en boucle dans un circuit calorifugé pour alimenter à nouveau le tube en tête, via une pompe.

Le flux d'acide acrylique technique est soumis à une série de plusieurs étages successifs de purification, chaque étage comprenant les étapes suivantes :
- cristallisation : le fluide caloporteur est refroidi rapidement de façon à abaisser la température du film tombant d'acide acrylique jusqu'à la température de cristallisation de l'acide acrylique dans le mélange, déterminée préalablement à partir d'un échantillon du mélange à purifier, puis un gradient négatif de température est imposé au fluide caloporteur, de 0, 1 à 0,5°C/min. Lorsque le volume d'acide acrylique cristallisé, mesuré par différence en évaluant le niveau de liquide dans le récipient collecteur en pied de cristalliseur, atteint 70% du mélange de départ, la recirculation du film tombant de mélange à purifier est stoppée, et le tube est égoutté. Le mélange liquide des eaux-mères obtenues ainsi est séparé et stocké dans un recette.
- ressuage : le fluide caloporteur est réchauffé de façon à provoquer la fusion d'une partie (5%) de la couche d'acide acrylique cristallisée à la surface du tube. Les eaux de cette étapes de ressuage sont collectées et stockées dans la même recette que les eaux-mères de l'étape précédente.
- fusion : le fluide caloporteur est rapidement réchauffé jusqu'à une température de 30°C jusqu'à fusion totale de la couche cristallisée. Le flux liquide purifié est placé dans une recette différente.

Le produit purifié par fusion dans la dernière étape du premier étage de purification est envoyé au deuxième étage de purification, où il sera soumis à une nouvelle série des 3 étapes de purification dans les mêmes conditions opératoires. Les eaux-mères du deuxième étage de purification sont ensuite mélangées avec une nouvelle charge du flux d'alimentation d'AA technique dans l'étage 1. Ce processus est répété ainsi jusqu'à obtenir la qualité désirée dans le produit purifié fondu.

Pour limiter les pertes d'acide acrylique concentrées dans les eaux-mères de premier étage de purification, on réalise une série d'étages successifs de concentration, présentant les mêmes étapes que les étages de purification, dans lesquels le cristallisat de l'étage "n-1" est envoyé en alimentation de l'étage "n" et les eaux-mères de cet étage "n-1" sont envoyées en alimentation de l'étage "n-2". Ces étages sont réalisés dans les mêmes conditions opératoires que les étages de purification, hormis le volume d'acide acrylique cristallisé visé, avant de passer de l'étape de cristallisation à l'étape de ressuage, qui est de 60% du produit alimenté.

Le dernier étage de cristallisation est réalisé en mode statique. Le flux à purifier est placé dans un récipient en inox à double paroi circulé par un fluide froid maintenu à la température de cristallisation du milieu, déterminée auparavant par une mesure de température de cristallisation. Dans ce récipient, on immerge un tube vertical en inox rempli de fluide caloporteur (mélange éthylèneglycol - eau) circulant en circuit fermé, via une pompe, à travers un échangeur de chaleur externe programmable en rampe de température.

Dans une première étape, on abaisse rapidement la température du fluide caloporteur dans le tube jusqu'à la température de cristallisation du milieu, puis on impose un gradient de température négatif de 0,1 à 0,5°C/min. Lorsque le volume cristallisé atteint environ 50% du produit de départ, on élimine les eaux-mères, puis on procède à une étape de ressuage et enfin à l'étape de fusion, comme dans les étages supérieurs de cristallisation en mode dynamique.

Appliqué à l'acide acrylique technique obtenu à partir de glycérol au terme des étapes de purification de l'exemple 3, une succession de 4 étages de purification et 3 étages de concentration dont un étage de cristallisation en mode statique a permis d'obtenir de l'acide acrylique de qualité "glacial" contenant 50 ppm d'acide acétique, 410 ppm d'acide propionique, moins de 1ppm d'anhydride maléique, moins de 80 ppm d'eau, moins de 1ppm d'acide 2-butènoïque, moins de 1ppm de furfural, moins de 1 ppm de benzaldéhyde, moins de 1 ppm de protoanémonine, moins de 1 ppm d'acroléine.

La concentration d'acide acrylique dans les eaux-mères résiduelles de l'étage ultime de concentration est de 82%.

Le rendement de récupération d'AA dans cette étape de purification est de 96,5%. Par ailleurs, le rendement global de récupération d'AA à partir du flux obtenu après l'étape (4) de séchage azéotropique est de 92%.

Avec un étage de concentration supplémentaire en mode dynamique, soit 4 étages de purification et 4 étages de concentration, dont l'un en mode statique, la concentration d'AA dans les eaux-mères ultimes est de 54,3 % et le rendement global de purification est de 99,3 %. Le résidu a la composition suivante en poids : AA : 54,3%, eau : 7,3% ; anhydride maléique : 8,9% ; protoanémonine : 1% ; benzaldéhyde : 2% ; acide acétique : 4,3% ; acide propionique : 16,7% ; acroléine : 1,6% ; furfural 0,8%: acide 2-butènoïque : 2%

### Exemple 5 : Purification de l'AA étêté ex-glycérol par cristallisation

Les mêmes séries de traitement avec un étage de cristallisation statique sont appliquées au flux obtenu en pied de la colonne d'étêtage (étape 5) de l'exemple 3.

Une succession de 4 étages de purification et 3 étages de concentration dont un étage de cristallisation statique a permis d'obtenir de l'acide acrylique de qualité "glacial" contenant moins de 50 ppm d'acide acétique, 500 ppm d'acide propionique, moins de 1 ppm d'anhydride maléique, moins de 100 ppm d'eau, moins de 1 ppm d'acide 2-butenoïque, moins de 1 ppm de furfural, moins de 1 ppm de benzaldéhyde, moins de 1 ppm de protoanémonine, moins de 1 ppm d'acroléine

La concentration d'acide acrylique dans les eaux-mères résiduelles de l'étage ultime de concentration est de 67%.

Le rendement de récupération d'AA dans cette étape de purification est de 97%. Par ailleurs, le rendement global de récupération d'AA à partir du flux obtenu après l'étape 4 de séchage azéotropique est de 94%.

### Exemple 6 : Purification de l'AA séché ex-glycérol par cristallisation

Les mêmes séries de traitement avec étage de cristallisation statique sont appliquées au flux obtenu en pied de la colonne de séchage (étape 4) de l'exemple 3.

Une succession de 4 étages de purification et 2 étages de concentration dont l'étage de cristallisation statique a permis d'obtenir de l'acide acrylique de qualité "glacial" contenant 1 200 ppm d'acide acétique, 450 ppm d'acide propionique, moins de 1 ppm d'anhydride maléique, moins de 100 ppm d'eau, moins de 1 ppm d'acide 2-butènoïque, moins de 1ppm de furfural, moins de 1 ppm de benzaldéhyde, moins de 1 ppm de protoanémonine, moins de 1 ppm d'acroléine

La concentration d'acide acrylique dans les eaux-mères résiduelles de l'étage ultime de concentration est de 62%

Le rendement global de récupération d'AA à partir du flux obtenu après l'étape 4 de séchage azéotropique est de 92%

### Exemple 7 (comparatif) : Purification de l'AA technique ex-glycérol par distillation

L'acide acrylique de grade technique issu de l'exemple 3 est additionné de phénothiazine (0.2%) et d'hydrate d'hydrazine dans un ratio molaire de 7/1 par rapport à la somme des aldéhydes (furfural, benzaldéhyde, acroléine) et de l'anhydride maléique présents et le flux est distillé dans une colonne de 17 plateaux perforés à déversoirs, sous une pression de 6,7.10³ Pa, avec une température de tête de 70°C et un taux de reflux de 0,5 / 1.

Le flux de distillat condensé est additionné d'une solution d'éther méthylique de l'hydroquinone (EMHQ) dans l'AA glacial (200 ppm par rapport à l'acide acrylique distillé).

Les analyses de l'acide acrylique obtenu montrent que le produit contient 0.07% d'acide acétique, 0.7% d'acide propionique, 3 ppm d'anhydride maléique, 0.7% d'eau, 75 ppm d'acide 2-butènoïque, moins de 1 ppm de furfural, moins de 1 ppm de benzaldéhyde, 38 ppm de protoanémonine, 2 ppm d'acroléine. Le taux de récupération d'acide acrylique de cette étape est de 93%.

Par ailleurs, au terme de cette dernière étape de purification pour obtenir la qualité d'AA glacial, le taux de récupération de l'AA à partir du flux de pied de colonne de séchage azéotropique de l'exemple 3 (étape (4)) n'est que de 86%.

L'acide acrylique produit selon de l'invention est un acide bio-ressourcé fabriqué à partir de matières premières naturelles non fossiles.

L'utilisation de matières premières carbonées d'origine naturelle non fossiles peut se détecter grâce aux atomes de carbone entrant dans la composition du produit final. En effet, à la différence des matières fossiles, les matériaux composés de matières premières renouvelables contiennent l'isotope radioactif ¹⁴C. Tous les échantillons de carbone tirés d'organismes vivants (animaux ou végétaux) sont en fait un mélange de 3 isotopes : ¹²C (représentant ∼ 98,892 %), ¹³C (∼ 1,108 %) et ¹⁴C (traces: 1,2.10⁻¹⁰ %). Le rapport ¹⁴C /¹²C des tissus vivants est identique à celui du CO₂ de l'atmosphère.

La constance du rapport ¹⁴C /¹²C dans un organisme vivant est lié à son métabolisme avec un continuel échange avec l'atmosphère.

La constante de désintégration du ¹⁴C est telle que la teneur en ¹⁴C est pratiquement constante depuis la récolte des matières premières végétales jusqu'à la fabrication du produit final..

L'acide acrylique bio-ressourcé obtenu par le procédé de l'invention a une teneur en masse de ¹⁴C telle que le ratio ¹⁴C /¹²C est supérieur à 0,8.10⁻¹² et de préférence supérieur à 1.10⁻¹².

La mesure de la teneur en ¹⁴C des matériaux est décrite précisément dans les normes ASTM D6866 (notamment D6866-06) et dans les normes ASTM D7026 (notamment 7026-04).

## Revendications

1. Procédé de fabrication d'acide acrylique bio-ressourcé à partir de glycérol comportant les étapes suivantes :
- déshydratation catalytique en phase gaz du glycérol en acroléine, (1)
- condensation partielle par refroidissement et extraction de l'eau contenue dans le milieu réactionnel de (1), (1')
- oxydation catalytique en phase gaz de l'acroléine en acide acrylique, (2)
- extraction de l'acide acrylique contenu dans l'effluent de l'oxydation par absorption à l'eau avec refroidissement, (3)
- séchage de la solution d'acide acrylique par distillation en présence d'un solvant non miscible à l'eau, (4)
- distillation de la solution ainsi obtenue pour éliminer les composés légers, (5)
- distillation de la fraction lourde issue de l'étape précédente (5) pour éliminer les composés lourds, (6)
combinées avec une étape finale d'extraction de l'acide acrylique par cristallisation fractionnée appliquée à l'un des effluents suivants : la fraction lourde de (4), fraction lourde de (5) ou fraction légère de (6).

2. Procédé selon la revendication 1 **caractérisé en ce que** la fraction liquide issue de (3) est soumise à une séparation (3') de l'acroléine résiduelle par distillation ou stripping avec un gaz.

3. Procédé selon l'une des revendications 1 ou 2 **caractérisé en ce que** la fraction lourde de (4) est soumise à l'étape finale d'extraction de l'acide acrylique par cristallisation fractionnée.

4. Procédé selon l'une des revendications 1 ou 2 **caractérisé en ce que** la fraction lourde de (5) est soumise à l'étape finale d'extraction de l'acide acrylique par cristallisation fractionnée.

5. Procédé selon l'une des revendications 1 ou 2 **caractérisé en ce que** la fraction légère de (6) est soumise à l'étape finale d'extraction de l'acide acrylique par cristallisation fractionnée.

6. Procédé selon l'une quelconque des revendications précédentes **caractérisé en ce que** l'étape de cristallisation fractionnée est effectuée selon la technique de la cristallisation fractionnée à film tombant.

7. Procédé selon la revendication 6 **caractérisé en ce que** l'étape de cristallisation fractionnée comprend entre 1 et 4 étages de purification et entre 1 et 4 étages pour la concentration des impuretés.

8. Procédé selon l'une des revendications 6 ou 7 **caractérisé en ce que** l'étape de cristallisation fractionnée est complétée par un étage de concentration par cristallisation statique.

## Patentansprüche

1. Verfahren zur Herstellung von aus biologischen Quellen stammender Acrylsäure aus Glycerin, das folgende Schritte umfasst:
- katalytische Dehydratisierung von Glycerin zu Acrolein in der Gasphase, (1)
- partielle Kondensation durch Abkühlung und Extraktion des in dem Reaktionsmedium von (1) enthaltenen Wassers, (1')
- katalytische Oxidation von Acrolein zu Acrylsäure in der Gasphase, (2)
- Extraktion der in dem Austragsstrom der Oxidation enthaltenen Acrylsäure durch Absorption mit Wasser unter Kühlung, (3)
- Trocknung der Acrylsäurelösung durch Destillation in Gegenwart eines nicht mit Wasser mischbaren Lösungsmittels, (4)
- Destillation der so erhaltenen Lösung zur Entfernung der leichten Verbindungen, (5)
- Destillation der schweren Fraktion aus dem vorhergehenden Schritt (5) zur Entfernung der schweren Verbindungen, (6)
kombiniert mit einem abschließenden Schritt der Extraktion von Acrylsäure durch fraktionierte Kristallisation eines der folgenden Austragsströme: der schweren Fraktion von (4), der schweren Fraktion von (5) oder der leichten Fraktion von (6).

2. Verfahren nach Anspruch 1, **dadurch gekennzeichnet, dass** die flüssige Fraktion aus (3) einer Abtrennung (3') des Restacroleins durch Destillation oder Strippen mit einem Gas unterworfen wird.

3. Verfahren nach einem der Ansprüche 1 oder 2, **dadurch gekennzeichnet, dass** die schwere Fraktion von (4) dem abschließenden Schritt der Extraktion der Acrylsäure durch fraktionierte Kristallisation unterworfen wird.

4. Verfahren nach einem der Ansprüche 1 oder 2, **dadurch gekennzeichnet, dass** die schwere Fraktion von (5) dem abschließenden Schritt der Extraktion der Acrylsäure durch fraktionierte Kristallisation unterworfen wird.

5. Verfahren nach einem der Ansprüche 1 oder 2, **dadurch gekennzeichnet, dass** die leichte Fraktion von (6) dem abschließenden Schritt der Extraktion der Acrylsäure durch fraktionierte Kristallisation unterworfen wird.

6. Verfahren nach einem der vorhergehenden Ansprüche, **dadurch gekennzeichnet, dass** der Schritt der fraktionierten Kristallisation gemäß der Technik der fraktionierten Fallfilmkristallisation durchgeführt wird.

7. Verfahren nach Anspruch 6, **dadurch gekennzeichnet, dass** der Schritt der fraktionierten Kristallisation zwischen 1 und 4 Reinigungsschritte und zwischen 1 und 4 Schritte zum Aufkonzentrieren der Verunreinigungen umfasst.

8. Verfahren nach einem der Ansprüche 6 oder 7, **dadurch gekennzeichnet, dass** der Schritt der fraktionierten Kristallisation durch einen Schritt des Aufkonzentrierens durch statische Kristallisation ergänzt wird.

## Claims

1. A process for the manufacture of bioresourced acrylic acid from glycerol, comprising the following stages:
- gas-phase catalytic dehydration of glycerol to give acrolein, (1)
- partial condensation by cooling and extraction of the water present in the reaction medium of (1), (1')
- gas-phase catalytic oxidation of the acrolein to give acrylic acid, (2)
- extraction of the acrylic acid present in the effluent stream from the oxidation by water absorption with cooling, (3)
- drying of the acrylic acid solution by distillation in the presence of a solvent that is immiscible with water, (4)
- distillation of the solution thus obtained in order to remove the light compounds, (5)
- distillation of the heavy fraction resulting from the preceding stage (5) in order to remove the heavy compounds, (6)
combined with a final stage of extraction of the acrylic acid by fractional crystallization applied to one of the following effluent streams: the heavy fraction from (4), the heavy fraction from (5) or the light fraction from (6).

2. The process as claimed in claim 1, **characterized in that** the liquid fraction resulting from (3) is subjected to a separation (3') from the residual acrolein by distillation or stripping with a gas.

3. The process as claimed in either of claims 1 and 2, **characterized in that** the heavy fraction from (4) is subjected to the final stage of extraction of acrylic acid by fractional crystallization.

4. The process as claimed in either of claims 1 and 2, **characterized in that** the heavy fraction from (5) is subjected to the final stage of extraction of acrylic acid by fractional crystallization.

5. The process as claimed in either of claims 1 and 2, **characterized in that** the light fraction from (6) is subjected to the final stage of extraction of acrylic acid by fractional crystallization.

6. The process as claimed in any one of the preceding claims, **characterized in that** the fractional crystallization stage is carried out according to the falling film fractional crystallization technique.

7. The process as claimed in claim 6, **characterized in that** the fractional crystallization stage comprises between 1 and 4 purification steps and between 1 and 4 steps for the concentration of the impurities.

8. The process as claimed in either of claims 6 and 7, **characterized in that** the fractional crystallization stage is supplemented by a step of concentration by static crystallization.
